# EUROPÄISCHE PATENTANMELDUNG

(11) **EP 0 878 188 A2**
(43) Veröffentlichungstag der Anmeldung: **18.11.1998**
(21) Anmeldenummer: 98108333.0
(22) Anmeldetag: 07.05.1998
(51) Int. Cl.: A61K 7/48, A61K 7/06

(54) **Verwendung von Milchsäureestern als Rückfettungsmittel in Haut- und Haarpflegemitteln**

(30) Priorität: 16.05.1997 DE 19720672
(71) Anmelder: Henkel Kommanditgesellschaft auf Aktien, 40589 Düsseldorf-Holthausen (DE)
(72) Erfinder: Seipel, Werner, 40723 Hilden (DE); Hensen, Hermann, Dr., 42791 Haan (DE); Rottmann, Mirella, 40231 Düsseldorf (DE); Franklin, Jutta, 40595 Düsseldorf (DE); Behler, Ansgar, Dr. Dipl.-Chem., 46240 Bottrop (DE)

(57) **Zusammenfassung**

Für die Herstellung von Rückfettungsmitteln wird der Einsatz von Milchsäureestern der Formeln **(Ia)**, **(Ib)** und/oder **(Ic)** vorgeschlagen, 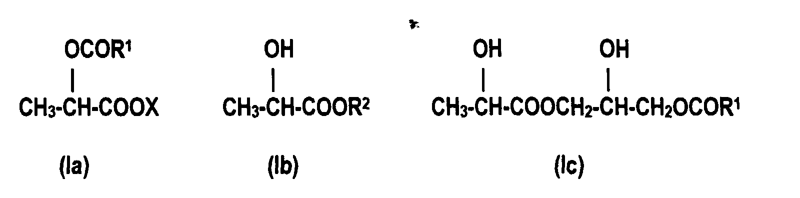 in denen R¹CO für einen linearen oder verzweigten, gesättigten und/oder ungesättigten Acylrest mit 6 bis 22 Kohlenstoffatomen, R² für einen Alkyl- und/oder Alkenylrest mit 6 bis 22 Kohlenstoffatomen und X für ein Alkali- und/oder Erdalkalimetall steht. Die Milchsäureester verbessern die sensorischen Eigenschaften von Haut und Haaren.

## Beschreibung

### Gebiet der Erfindung

Die Erfindung betrifft die Verwendung von Milchsäureestern als Rückfettungsmittel zur Herstellung von Haut- und Haarpflegemitteln.

### Stand der Technik

Bei der Reinigung Von Haut und Haaren wird nicht nur anhaftender Schmutz entfernt, bei der wiederholten Anwendung kommt es auch zum Abbau des natürlichen Lipidfilms, was sich nachteilig in einem stumpfen, spröden Hautgefühl bzw. in einer erschwerten Kämmbarkeit der Haare bemerkbar macht. Aus diesem Grunde werden Haut- und Haarpflegemitteln gewöhnlich Rückfettungsmittel wie beispielsweise ethoxylierte Lanolinderivate zugesetzt, die den Ölfilm wieder ergänzen Aus Sicht des Verbrauchers wie Kosmetikchemikers besteht jedoch ein Bedarf an Rückfettungsmitteln, die gegenüber den bekannten Wirkstoffen nicht nur verbesserte hautkosmetische Eigenschaften aufweisen und in diesem Zusammenhang vor allem frei von Ethylenoxid sind, sondern auch in ihrem Leistungsspektrum Vorteile aufweisen. In diesem Zusammenhang wären beispielsweise solche Produkte besonders attraktiv, die rückfettende mit emulgierende Eigenschaffen verbinden. Die Aufgabe der Erfindung hat darin bestanden, solche Stoffe zur Verfügung zu stellen.

### Beschreibung der Erfindung

Gegenstand der Erfindung ist die Verwendung von Milchsäureestern der Formeln **(Ia)**, **(Ib)** und/oder **(Ic)**, 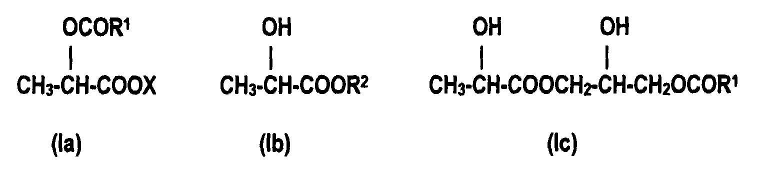 in denen R¹CO für einen linearen oder verzweigten, gesättigten und/oder ungesättigten Acylrest mit 6 bis 22 Kohlenstoffatomen, R² für einen Alkyl- und/oder Alkenylrest mit 6 bis 22 Kohlenstoffatomen und X für ein Alkali- und/oder Erdalkalimetall steht, als Rückfettungsmittel zur Herstellung von Haut- und Haarpflegemittel.

Übetraschenderweise wurde gefunden, daß Milchsäureester nicht nur über emulgierende Eigenschaften verfügen, sondern auch als Rückfettungsmittel Haut und Haaren einen angenehmen Weichgriff verleihen. Bei der Anwendung in Haarpflegemitteln wird zudem eine Verbesserung der Kämmbarkeit erzielt.

### Milchsäureester

Milchsäureester stellen bekannte Emulgatoren des Stands der Technik dar, die in der Regel durch Umsetzung von Milchsäure mit Fettsäuren, Fettalkoholen oder Glycerin erhalten werden. In Abhängigkeit der Menge an Einsatzstoffen läßt sich ein definierter Gehalt an gebundener Milchsäure in den Veresterungsprodukten einstellen [vgl. A.M.Chaudhry et al. in **Pak.J.Sci.Res. 33, 344 (1990)**.] Im Sinne der Erfindung kommen als Milchsäureester, die der Formel **(Ia)** folgen, Veresterungsprodukte von Milchsäure mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Etucasäure sowie deren technische Mischungen in Frage, die in Form ihrer Alkali- und/oder Erdalkalisalze vorliegen können. Bevorzugt ist der Einsatz von Milchsäureestern der Formel **(Ia)**, bei denen sich der Acylrest R¹CO von Fettsäuren mit 16 bis 22 Kohlenstoffatomen ableitet, wie beispielsweise das Natriumsalz des Stearoyllactats (Sodium Stearoyl Lactate).

Neben Estern, die durch Acylierung der Hydroxylgruppe der Milchsäure erhalten werden, kommen auch Ester in Frage, die der Formel **(Ib)** folgen und Veresterungsprodukte der Milchsäure mit Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylakoho, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen darstellen. Bevorzugt ist der Einsatz von Milchsäureestern der Formel **(Ib)**, bei denen sich der Alkylrest R² von Fettalkoholen mit 16 bis 22 Kohlenstoffatomen ableitet, wie beispielsweise Milchsäurestearylester.

Als dritte Gruppe von Milchsäureestern kommen Partialglyceride der Formel **(Ic)** in Frage, die beispielsweise durch Veresterung der Milchsäure mit technischen Partialglyceriden erhalten werden.

Dabei entstehen in der Regel technische Gemische, in denen auch Estolide und Lactide - dies trifft auch für die Ester der Formeln **(Ia)** und **(Ib)** zu - enthalten sein können. Die Partialglyceride ihrerseits können Mono- und/oder Diester des Glycerins mit Fettsäuren mit 6 bis 22 und vorzugsweise 12 bis 18 Kohlenstoffatomen darstellen.

Allen diesen Estertypen ist gemeinsam, daß sie in einer besonders bevorzugten Ausführungsform der Erfindung einen Veresterungsgrad im Bereich von 50 bis 90 und vorzugsweise 60 bis 80 % aufweisen. Die Milchsäureester können dabei in Mengen von 1 bis 10, vorzugsweise 3 bis 7 Gew.-% - bezogen auf die Zubereitungen - eingesetzt werden.

### Gewerbliche Anwendbarkeit

Die Milchsäureester können im Sinne der Erfindung zur Herstellung der Haut- und Haarpflegemittel zusammen mit weiteren Hilfs- und Einsatzstoffen, wie z.B. milden Tensiden, Ölkörpern, Emulgatoren, Überfettungsmitteln, Stabilisatoren, Wachsen, Konsistenzgebern, Verdickungsmitteln, Kationpolymeren, Siliconverbindungen, biogene Wirkstoffen, Antischuppenmitteln, Filmbildnern, Konservierungsmitteln, Hydrotropen, Solubilisatoren, UV-Lichtschutzfiltern, Insektenrepellentien, Selbstbräunern, Farb- und Duftstoffen und dergleichen verwendet werden.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate Guerbetcarbonate, Dialkylether, Ringöffnungsprodukten von epoxidierten Fettsäureestern mit Polyolen, Siliconöle und/oder aliphatische bzw. naphthenische Kohlenwasserstoffe in Betracht.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckenest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquatemierte Difettsäuretriethanolaminester-Salze, besonders bevorzugt sind.

Als weitere **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. ein quatemierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere wie z.B. Luviquat® (BASF), Kondensationsprodukte von Polyglycolen und Aminen, quaternierte Kollagenpolypeptide wie beispielsweise Lauryldimonium hydroxypropyl hydrolyzed collagen (Lamequat®L/Grünau), quaternierte Weizenpolypeptide, Polyethylenimin, kationische Siliconpolymere wie z.B. Amidomethicone, Copolymere der Adipinsäure und Dimethylaminohydroxypropyldiethylentrimamin (Cartaretine®/Sandoz), Copolymere der Acrylsäure mit Dimethyldiallylammoniumchlorid (Merquat® 550/Chemviron), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese, quaternierte Ammoniumsalz-Polymere wie z.B. Mirapol® A-15, Mirapol® AD-1, Mirapol® AZ-1 der Miranol.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor-, glucosid- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Perlglanzwachse** können insbesondere Mond und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zink-stearat eingesetzt werden. Unter **biogenen Wirkstoffen** sind beispielsweise Tocopherol, Tocopherol-acetat, Tocopherolpalmitat, Ascorbinsäure, Retinol, Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinyl-pyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. Typische Beispiele sind 4-Aminobenzoesäure sowie ihre Ester und Derivate (z.B. 2-Ethylhexyl-p-dimethylarninobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoylmethane, Salicylatester, 2-Phenylbenzimidazol-5-sulfonsäure, 1-(4-tert.Butylphenyl)-3-(4'-methoxyphenyl)-propan-1,3-dion, 3-(4'-Methyl)benzylidenbornan-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können ferner **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage, als **Selbstbräuner** eignet sich Dihydroxyaceton. Als **Farbstoffe** können die für kosmetische Zwecke geeigneten und zugelassenen Substanzen verwendet werden, wie sie beispielsweise in der Publikation **"Kosmetische Farbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

Zur Untersuchung des rückfettenden Verhaltens wurde die Naßkämmbarkeit von Milchsäureestern behandelter Haarsträhnen untersucht. Hierzu wurden die Strähnen vor der Nullmessung mediumblondiert. Nach einer Einwirkzeit von 5 min wurden die Testformulierungen (1g/1g Haar) unter Standardbedingungen (38°C, 1L/min) 1 min gespült. Die Messung wurde an 20 Haarsträhnen durchgeführt. Die Ergebnisse sind in Tabelle 1 zusammengefaßt:

**Tabelle 1**

| **Naßkämmbarkeitsmessungen (Mengenangaben als Gew.-%)** | | | | | | |
|---|---|---|---|---|---|---|
| **Zusammensetzung/Performance** | **1** | **2** | **3** | **4** | **5** | **6** |
| Sodium Laureth Sulfate | 15,0 | | | | | |
| Sodium Caproyllactylat | 5,0 | - | - | - | - | - |
| Sodium Lauroyllactylat | - | 5,0 | - | - | - | - |
| Sodium Cocoyllactylat | - | - | 5,0 | - | - | - |
| Sodium Stearoyllactylat | - | - | - | 5,0 | - | - |
| Sodium Stearoyllactylat | - | - | - | - | 5,0 | - |
| Sodium Stearoyllactylat | - | - | - | - | - | 5,0 |
| Wasser | ad 100 | | | | | |
| ***Veresterungsgrad Milchsäureester*** | 18 | 36 | 24 | 34 | 67 | 75 |
| ***Naßkämmbarkeit, vorher [mJ]*** | 67,7 | 65,1 | 60,0 | 60,3 | 62,4 | 63,5 |
| ***Naßkämmbarkeit, nachher [mJ]*** | 57,1 | 61,7 | 57,5 | 53,7 | 37,3 | 41,0 |
| ***Rest [%]*** | 84 | 95 | 96 | 89 | 60 | 65 |

## Patentansprüche

1. Verwendung von Milchsäureestern der Formeln **(Ia)**, **(Ib)** und/oder **(Ic)**, 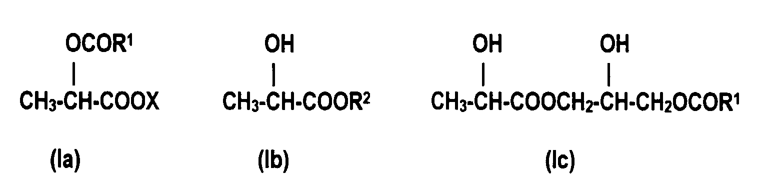 in denen R¹CO für einen linearen oder verzweigten, gesättigten und/oder ungesättigten Acylrest mit 6 bis 22 Kohlenstoffatomen, R² für einen Alkyl- und/oder Alkenylrest mit 6 bis 22 Kohlenstoffatomen und X für ein Alkali- und/oder Erdalkalimetall steht, als Rückfettungsmittel zur Herstellung von Haut- und Haarpflegemiteln.

2. Verwendung nach Anspruch 1, **dadurch gekennzeichnet**, daß man Milchsäureester der Formel **(Ia)** einsetzt, bei denen sich der Acylrest R¹CO von Fettsäuren mit 16 bis 22 Kohlenstoffatomen ableitet.

3. Verwendung nach den Ansprüchen 1 und 2, **dadurch gekennzeichnet**, daß man Milchsäureester der Formel **(Ib)** einsetzt, bei denen sich der Alkylrest R² von Fettalkoholen mit 16 bis 22 Kohlenstoffatomen ableitet.

4. Verwendung nach den Ansprüchen 1 bis 3, **dadurch gekennzeichnet**, daß man Milchsäureester einsetzt, die einen Veresterungsgrad im Bereich von 50 bis 90 % aufweisen.
